# EUROPÄISCHE PATENTSCHRIFT

(11) **EP 2 338 560 B1**
(45) Veröffentlichungstag und Bekanntmachung des Hinweises auf die Patenterteilung: **04.11.2015**
(21) Anmeldenummer: 10192881.0
(22) Anmeldetag: 29.11.2010
(51) Int. Cl.: A61N 1/08, A61N 1/37, A61N 1/39

(54) **Implantierbarer Kardioverter-Defibrillator (ICD) mit MRT-Störerkennungseinheit**
Implantable cardioverter defibrillator (ICD) with MRI interference detection unit
Défibrillateur cardioverseur implantable (ICD) avec unité de détection d'interférence IRM

(30) Priorität: 22.12.2009 US 288854 P
(43) Veröffentlichungstag der Anmeldung: 29.06.2011
(73) Patentinhaber: Biotronik CRM Patent AG, 6341 Baar (CH)
(72) Erfinder: Dörr, Thomas, 12437 Berlin (DE)
(74) Vertreter: Galander, Marcus

(56) Entgegenhaltungen:
- EP-A1- 1 935 450
- WO-A1-96/41203
- US-A- 6 101 417
- US-A1- 2003 144 704
- US-A1- 2004 088 012
- US-A1- 2006 293 591

## Beschreibung

Die Erfindung bezieht sich auf eine Vorrichtung und ein Verfahren zur Erkennung von elektromagnetischen Feldern, speziell solche Felder, wie sie bei bildgebenden Kernspintomographie-Geräten (im folgenden MRT- oder MRI-Geräte) auftreten.

Obwohl MRT-Untersuchungen einen immer höheren Stellenwert in der diagnostischen Medizin erhalten, ist ein Teil der Patienten für MRT-Untersuchungen kontraindiziert. Eine solche Kontraindikation kann durch ein aktives implantiertes medizinisches Gerät (im Folgenden auch Implantat oder IMD) gegeben sein. Neben den MRI-Untersuchungen stellen aber auch andere technische Anwendungen eine Gefahr für die Nutzer von medizinischen Geräten oder implantierbaren medizinischen Geräten dar, besonders wenn diese starke elektromagnetische Störfelder (EMI, Electro Magnetic Interference) in Ihrer Umgebung erzeugen.

Um MRT-Untersuchungen dennoch zu ermöglichen, sind verschiedene Ansätze, die sich entweder auf die Durchführung der MRT-Untersuchung oder auf das implantierbare medizinische Gerät beziehen, bekannt.

Unter anderem sind Technologien zur Erkennung von Magnetfeldern bekannt, die auf herkömmlichen Verfahren zur Magnetfelddetektion beruhen. So beschreibt die US 2008/0154342 ein Verfahren unter Ausnutzung eines GMR-Sensors (Giant Magnetic Resistance), um problematische Magnetfelder von MRT-Geräten zu erkennen.

Des Weiteren beschreibt die US 7,164,950 einen Ansatz zur Detektion von MRT typischen Störfeldern, der auf die Detektion von RF-Feldern (Radio Frequency) mittels zusätzlicher Antennen abzielt, allerdings ist in diesem Zusammenhang immer ein weiterer Sensor zur Detektion von Magnetfeldern notwendig. Damit besitzt das im Stand der Technik beschriebene System mehre Nachteile, wie die Abschaltung von relevanten Funktionen, wie zum Beispiel der antibradykarden Stimulation, das Vorhanden sein von gefilterten und ungefilterten Durchführungen in das Implantat und eine notwendige Anpassung des RF-Detektors mittels eines Magnetfeldsensors.

Die EP1935450A1 und die WO96/41203 beschreiben Vorrichtungen um RF-Felder im Umfeld eines Implantates zu erkennen.

Daher ist es Aufgabe der vorliegenden Erfindung, eine einfache und zuverlässige Vorrichtung und Methode zur Erkennung von typischen MRT-Feldern für ein IMD bereitzustellen. Die Aufgabe wird von einem implantierbaren Kardioverter-Defibrillator (ICD) nach Anspruch 1 gelöst.

Des ICD nach Anspruch 1 umfasst dabei mindestens:
- ein hermetisch abgeschlossenen Gehäuse,
- mindestens eine Steuereinheit,
- mindestens eine RF-Antenne
- eine RF-Kommunikationseinheit, wobei die RF-Kommunikationseinheit in Verbindung mit der RF-Antenne ausgestaltet ist, eine Kommunikation zwischen einem externen Programmiergerät und dem implantierten medizinischen Gerät zu ermöglichen, einen Hochspannungskondensator und eine MRT-Störerkennungseinheit mit einer Demodulationseinheit,
   wobei die MRT-Störerkennungseinheit mittels mindestens einer Frequenzweiche mindestens mit der RF-Kommunikationseinheit und/oder der RF-Antenne verbunden ist und die Demodulationseinheit die MRT typischen RF-Drehfelder erkennt und ein MRT-Erkennungssignal von der MRT-Erkennungseinheit an die mindestens eine Steuereinheit übermittelt wird, wobei die MRT-Störerkennungseinheit nur vor oder nur beim Laden des Hochspannungskondensators eine MRT-Erkennung durchführt.

In einer bevorzugten Ausführung ist die RF-Antenne für eine Kommunikation im MICS-(medical implant communication service) Band und/oder ISM- (Industrial, Scientific and Medical) Band ausgebildet.

Auch bevorzugt ist, dass beim Erkennen eines MRT dies in einem Diagnostikspeicher notieren vermerkt wird und/oder während einer MRT-Untersuchung keine Episoden aufgezeichnet werden. So kann verhindert werden, dass eine MRT-Sitzung die Diagnostikdaten mit Artefakten überschreibt.

Bevorzugt handelt es sich bei dem IMD um einen Herzschrittmacher und/oder ICD (Implanted Cardioverter-Defibrillator) und/oder ein CRT-Gerät (Cardiac Resynchronization Therapy-Gerät) und/oder Neurostimulator und/oder ein Implantat zur Überwachung von physiologischen Signalen, wie, aber nicht beschränkt auf, einen implantierten Monitor zur Überwachung der Herzfunktion.

In einer weiteren bevorzugten Ausführung beruht die MRT-Erkennung auf einer Auswertung des MTR-typischen Frequenzspektrums an der Antenne.

In einer bevorzugten Variante werden die für unterschiedliche Gerätetypen typischen Magnetfeldstärken typischen Frequenzen zugeordnet. So entsteht für die typischen Frequenzen ein Parameterraum von etwa 64 MHz für 1,5 Tesla Geräte bis zu 300 MHz für 7,0 Tesla Geräte.

Außerdem ist bevorzugt, dass die MRT-Störerkennungseinheit über die eine Frequenzweiche zusätzlich mit einer vorhandenen Elektrodenleitung verbunden und/oder verbindbar ist und eine MRT-Erkennung nur vorliegt, wenn RF-Drehfelder gleichzeitig sowohl über die Antenne als auch die Elektrodenleitung erkannt werden.

Auch ist bevorzugt, dass das Antennendiagramm der RF-Antenne in drei Raumrichtungen Maxima aufweist, bevorzugt in drei orthogonale Raumrichtungen. Dadurch kann ein Drehfeld von einem gepulsten Wechselfeld in der Demodulationseinheit unterschieden werden. Hierfür können neben einer typischen RF-Antenne auch weitere RF-Antennen vorhanden sein, um eine hohe Signifikanz bezüglich der Unterscheidung von Drehfeldern und gepulsten Wechselfeldern zu erreichen.

Bevorzugt ist auch, dass bei einer Erkennung eines MRT-typischen Feldes in einen MRT-sicheren Zustand gewechselt wird, wobei dieser Zustand entweder bis zu einer möglichen Umprogrammierung permanent oder für einen vorbestimmten Zeitraum befristet oder bis zu einem Ausbleiben einer MRT-Erkennung oder bis zu einem Ausbleiben einer MRT-Erkennung für einen vorbestimmten Zeitraum beibehalten wird. Eingeschlossen sind dabei auch logische Verknüpfungen der genannten Zeiträume für das Beibehalten des MRT-sicheren Zustands, insbesondere Kombinationen, bei denen ein jeweils frühestes oder spätestes Ereignis den Zeitraum bestimmt. Die Regeln für die Beibehaltung des MRT-sicheren Zustands sind vorbestimmt oder vorbestimmbar. Durch eine patienten- und untersuchungsabhängige Vorbestimmung kann ein für den jeweiligen Patienten optimaler Betrieb des IMD gewährleistet werden, ohne das Gerät und/oder den Patienten zusätzlichen Risiken durch die MRT-Untersuchung auszusetzen.

In einer weiteren bevorzugten Ausführung weist der MRT-sichere Zustand eine vorgegebene oder vorbestimmte Stimulationscharakteristik auf.

Des Weiteren ist bevorzugt, dass die Antenne während der MRT-Erkennung auf die typischen MRT-RF-Frequenzspektren abgestimmt wird, wobei eine feste Frequenz vorgegeben sein kann oder ein oder mehrere Frequenzbereiche abgetastet werden.

Insbesondere sind die typischen Frequenzbereiche zwischen 64 MHz für 1,5 Tesla MRT-Geräte und 300 MHz für 7,0 Tesla Geräte bevorzugt, die Frequenzbereiche lassen sich aber auch auf andere Bereiche erweitern, speziell wenn andere Magnetfeldstärken Anwendung finden.

Auch ist bevorzugt, dass die Abtastung von einem oder mehreren Frequenzbereichen entweder über einen oder mehrere Bandpassfilter oder programmierbare Bandpassfilter erfolgt.

In einer weiteren bevorzugten Ausführung werden für MRT-Geräte, die mit unterschiedlichen Magnetfeldstärken und damit unterschiedlichen RF-Frequenzen arbeiten, unterschiedliche vorbestimmte MRT-sichere Zustände automatisch ausgewählt.

Bevorzugt ist auch, dass das IMD in einen MRT-sicheren Zustand geschaltet wird, wenn die MRT-Störerkennungseinheit ein MRT-Feld erkannt hat und dass der MRT-sichere Zustand eine um eine vorbestimmte Zeit verlängerbare VF-Detektion hat, um eine sichere Unterscheidung zwischen normalen RF-Störungen und denen eines MRT zu unterscheiden. Unter VF (ventricular fibrillation) sind in diesem Zusammenhang alle schnellen Herzrhythmusstörungen zu verstehen, wobei für jeden Patienten eine individuelle angemessene Grenzfrequenz anzunehmen und/oder zu bestimmen ist oder eine vorbestimmte Grenzfrequenz gewählt wird. VF steht somit stellvertretend für die Wahrnehmung und Klassifikation einer anhaltenden tachykarden ventrikulären Rhythmusstörung, die in der Regel als therapiepflichtig eingestuft ist und mittels antitachykarder Stimulation oder Defibrillationsschocktherapie vom ICD behandelt wird.

Besonders bevorzugt wird, dass die MRT-Störerkennungseinheit des Weiteren mit weiteren Indikatoren für MRT-Störfelder oder mindestens einem MRT-Sensor verbunden ist. Die MRT-Erkennung beruht dabei auf der Erkennung durch mindestens einen der Sensoren oder Indikatoren. Unter MRT-Sensor oder MRT-Indikator sind im Zusammenhang mit dieser Anmeldung neben der erfindungsgemäßen MRT-Erkennung alle Sensoren oder Geräte oder Bauteile zu verstehen, die eine Erkennung von MRT-Feldern oder anderen starken elektromagnetischen Feldern ermöglichen. Dazu gehören unter anderen, aber nicht beschränkt auf diese, GMR-Sensoren, MagFET-Sensoren, Hallsensoren, die Überwachung von Batteriespannungen während Kondensatorladeprozessen, die Detektion von Gradientenfeldern, die Detektion von durch elektromagnetische Felder induzierten Strömen, die Detektion durch Leuchtdioden, die von MRT-Feldern zur Lichtemission angeregt werden, die Detektion von spezifischen Vibrationen oder als Sensoren ausgelegte Bauteile zur Detektion von durch Lorentzkräfte induzierten Vibrationen. Des Weiteren kann auch ein Lagesensor, speziell ein selbstkalibrierender Lagesensor, zur Erhöhung der Spezifität der MRT-Erkennung herangezogen werden.

Des Weiteren wird bevorzugt, dass ein Lagesensor zur Plausibilitätsprüfung herangezogen wird und eine positive MRI-Erkennung nur erfolgt, wenn der Lagesensor eine liegende Positur und/oder eine andere voreinstellbare Positur meldet.

Besonders bevorzugt ist der Lagesensor selbst kalibrierend, wobei die Kalibrierung unter voreinstellbaren Randbedingungen, wie, aber nicht beschränkt auf, Uhrzeiten und/oder Herzraten und/oder Atemfrequenz und/oder hämodynamische Parameter und/oder Aktivität (Bewegungssensor) stattfindet.

Auch ist bevorzugt, dass mindestens eine der folgenden Maßnahmen bei einer MRT-Erkennung eingeleitet wird:
- das Wechseln in einen MRT-sicheren Zustand
- ein verlängertes Verbleiben in einem MRT-sicheren oder gegenüber elektromagnetischen Störfeldern unempfindlichen Zustand,
- eine Synchronisierung von elektrischen Messungen (z. B. Impedanzmessungen) mit bei periodischen oder gepulsten elektromagnetischen Feldern auftretenden betragsmäßigen Feldstärkenminima oder die Synchronisierung einer Stimulation mit eben jenen betragsmäßigen Minima, und
- die Abgabe von elektromagnetischen Pulsen zur Signalisierung, dass ein medizinisches Gerät, speziell ein Implantat, im elektromagnetischen Feld vorhanden ist, speziell zur Signalisierung an ein MRT-Gerät, mit der Möglichkeit neben der Störung auch Informationen auf diese Weise zu übertragen und auf dem Bildschirm des MRT sichtbar zu machen.

### Beschreibung der Figuren:

- Fig. 1: Schematische Darstellung des Ablaufs einer MRT-Untersuchung.
- Fig. 2: Blockschaltbild eines erfindungsgemäßen IMD mit MRT-Erkennungseinheit
- Fig. 3: Blockschaltbild eines erfindungsgemäßen IMD mit MRT-Erkennungseinheit
- Fig. 4: Blockschaltbild eines erfindungsgemäßen IMD mit MRT-Erkennungseinheit

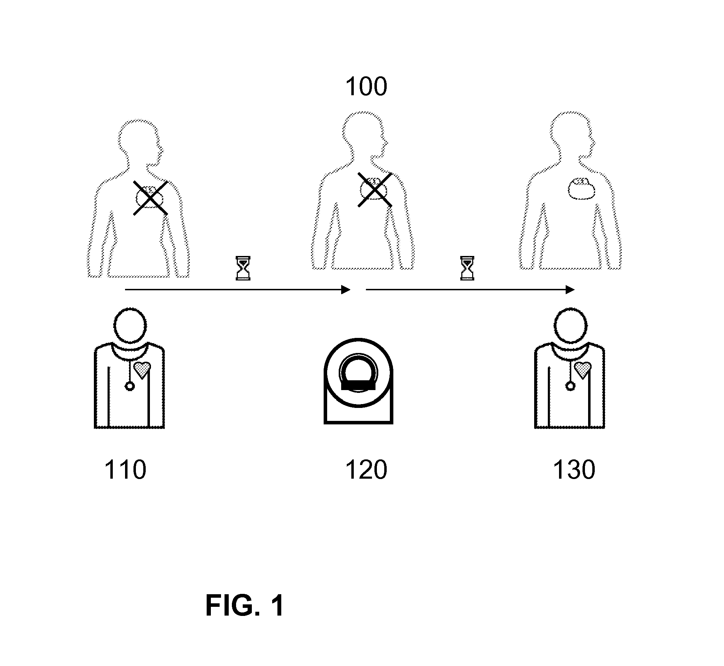

In der Figur 1 ist die Ausgangssituation, das heißt der derzeitige klinische Stand der Technik, dargestellt. Der ICD-Patient (100) wird vor der geplanten MRT-Untersuchung von einem Kardiologen nachgesorgt und der ICD ausgeschaltet (110). Mit einer zeitlichen Verzögerung von Stunden bis Tagen erfolgt die MRT-Untersuchung bei einem Radiologen (120). Nach einer weiteren Verzögerung wird der Patient wieder vom Kardiologen (130) betreut und der ICD wieder eingeschaltet. Während der gesamten Zeit von (110) bis (130) ist der Patient ohne den Schutz des implantierten Defibrillators und weitgehend ohne Rhythmusmonitoring. Derzeit wird dieses verbleibende Restrisiko, gemessen an dem Nutzen der MRT-Untersuchung in Kauf genommen. Ebenso ist der finanzielle und logistische Aufwand einer solchen Prozedur sehr hoch und schließt in vielen Fällen die Notfallanwendung eines MRT aus. Ähnlich wird bei Schrittmacherpatienten und Patienten mit anderen Implantaten verfahren, wobei die Geräte nicht zwangsläufig in einen inhibierten Modus versetzt werden, sondern auch andere Betriebsmodi patientenindividuell verwendet werden. Gemeinsam ist jedoch allen Prozessen, dass der Patient im Vorfeld, während und nach der MRT-Untersuchung nicht optimal versorgt ist.

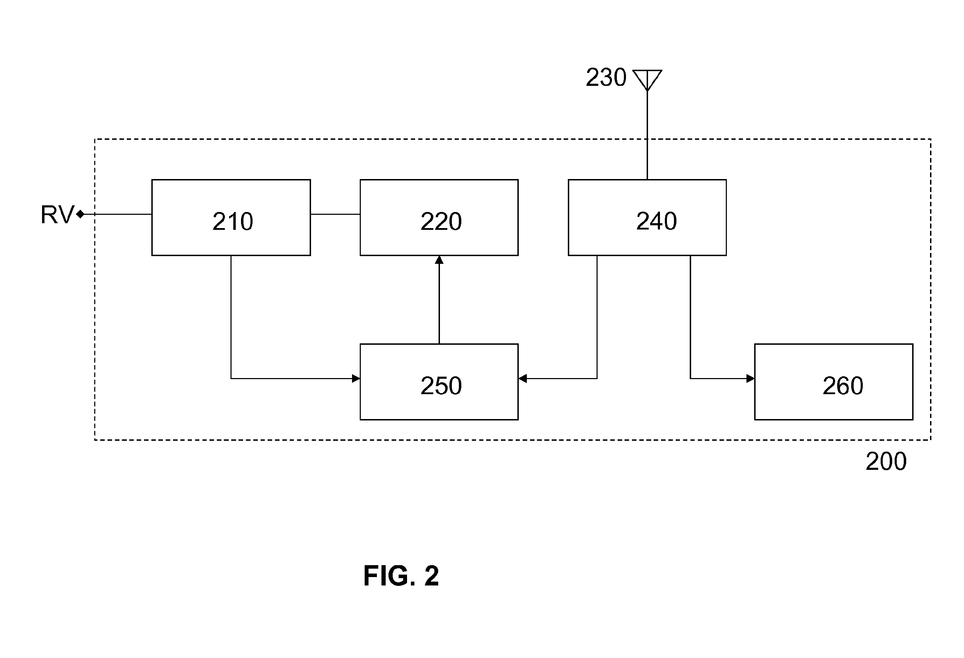

Die Figur 2 zeigt das Blockschaltbild der erfindungsgemäßen Lösung zur Detektion des MRT-Störfeldes im elektronischen Implantat (200) am Beispiel eines ICD (ebenso sind IPG, Neurostimulatoren, Medikamentenpumpen o. ä. möglich.
Die zur Detektion von ventrikulärem Flimmern (VF) eingesetzte rechtsventrikuläre Elektrode (RV) ist mit einer Einheit zur EKG-Signalwahrnehmung/Rhythmusklassifikation und Stimulation (210) verbunden. Diese wiederum ist mit einer Therapiesteuereinheit (220) verbunden, die entsprechend der Rhythmusklassifikation die entsprechende Stimulationstherapie auswählt. Zur Kommunikation mit dem Programmiergerät verfügt das Implantat über eine RF-Antenne (230). Diese wird erfindungsgemäß über eine Frequenzweiche zum einem mit der RF-Telemetrieeinheit (260) verbunden und zum anderen mit einem Störsignaldetektor (250). Dieser Störsignaldetektor (250) ist außerdem mit der EKG-Signalwahrnehmungseinheit (210) verbunden. Der Störsignaldetektor ist derart ausgeführt, dass der das zeitgleiche Auftreten von hochfrequenten Signalanteilen an der Elektrode (RV) und der RF-Antenne (230) von hochfrequenten Signalen an nur einer Signalquelle differenzieren kann. Die Hochfrequenz der RF-Antenne wird dabei durch eine Bandpassfilterung auf die typischen Frequenzen des MRT abgestimmt und anschließend dem Drehfelddemodulator (270) zugeführt. Dieser Drehfelddemodulator (270) liefert immer dann ein Ausgangssignal, wenn die dem Drehfeld typische Modulation demoduliert und klassifiziert wurde (z. B. Amplitudendemodulation). Nimmt der Störsignaldetektor (250) ein gleichzeitiges Einsetzen der Störung an der Elektrode (RV) und ein erkanntes Drehfeld an der Antenne (230) wahr, so signalisiert dieser der Steuereinheit das wahrscheinliche Vorhandensein eines MRT in der Umgebung des Implantates. In diesem Fall wird die Steuereinheit automatisch eine zuvor festgelegtes (programmierte) Parameterkombination für den sichern Betrieb innerhalb eines MRT einstellen (z. B. V00-Mode und VF-Detektion aus).

Eine alternative Implementierung des MRT-Störsignaldetektors (250) wertet zusätzlich die Amplitude des bandpassgefiltertem Störsignals von der RF-Antenne (230) im vordefinierten typischen Frequenzbereich des MRT aus.

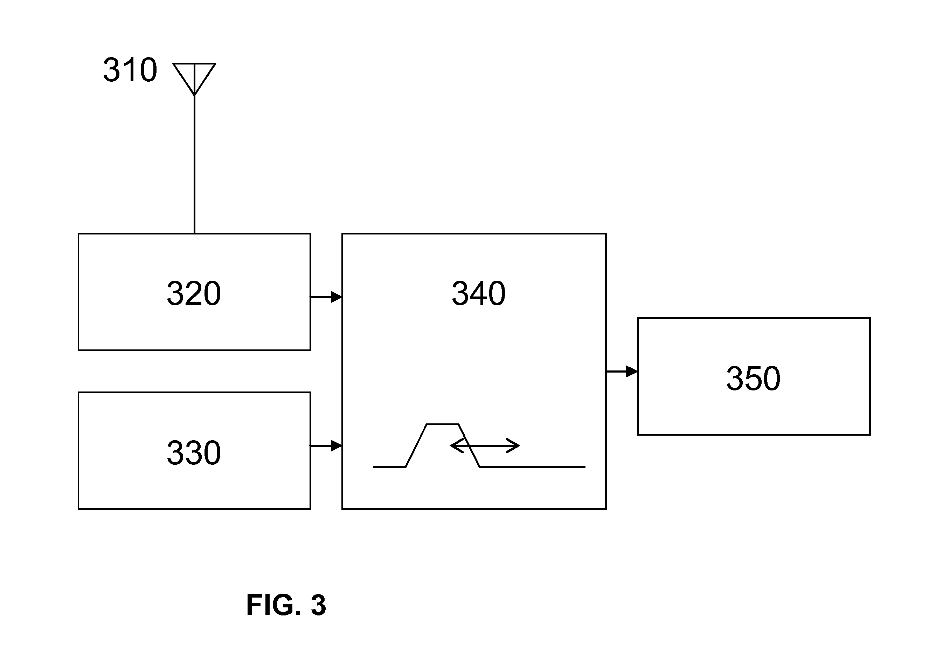

In der Figur 3 ist eine Erweiterung des Blockschaltbildes aus Figur 2 dargestellt. Hier ist die RF-Antenne (310) ebenfalls mit einer Frequenzweiche (320) verbunden. Deren Ausgangssignal für die MR-RF-Felderkennung wird einem programmierbaren Filter (340) zugeführt. Dieser programmierbare Bandpassfilter (340) kann über eine Steuereinheit (330) hinsichtlich der Eckfrequenzen umprogrammiert werden, so dass ein sequenzieller Scan aller möglichen Frequenzbereiche verschiedener MRT-Anlagen (z. B. von 1,5T ... 7T = ~64MHz ... -298 MHz) ermöglicht wird. Der Ausgang des programmierbaren Filters (340) ist immer mit dem Drehfelddemodulator (350) verbunden.

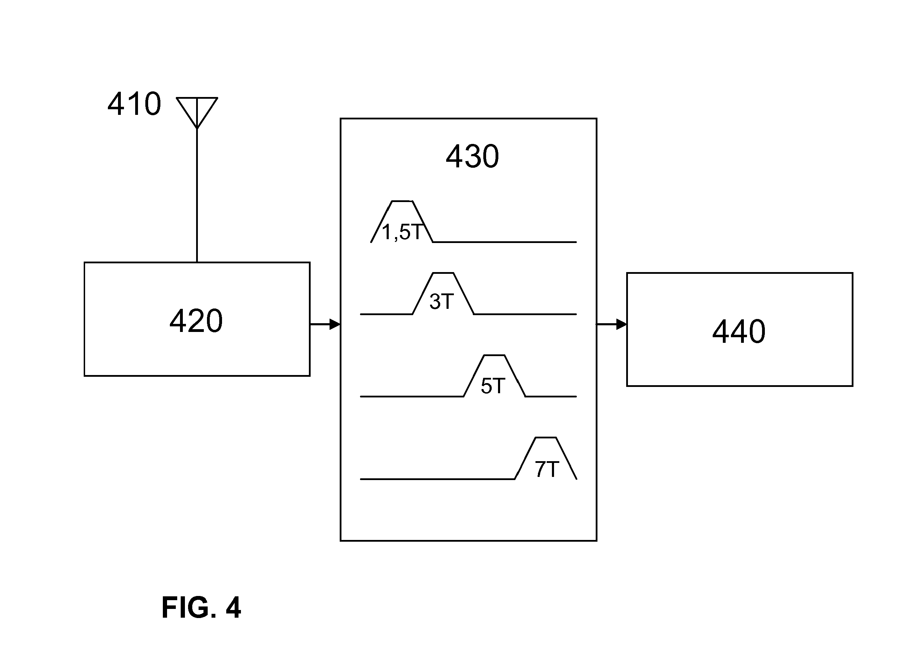

In der Figur 4 ist optional der Einsatz eines mehrkanaligen Filters (430) dargestellt. Die RF-Antenne (410) wird mit diesem Filterblock über eine Frequenzweiche (420) verbunden. Der mehrkanalige Filter ist so ausgelegt, dass er jeweils einen Bandpass für die jeweils gängigen MRT-RF-Frequenzen enthält. Der Ausgang dieses Filters wird dann immer mit dem Drehfelddemodulator (440) verbunden. Um eine unspezifische Deaktivierung der VF-Detektion über einen längeren Zeitraum zu vermeiden, ist es optional möglich, dass die Störsignalerkennung per Programmierung auf ein bestimmtes Zeitfenster per Programmierung beschränkt wird. So kann bei einer planmäßigen MRT-Untersuchung z. B. die MRT-Störerkennung für einige Tage per Programmierung aktiviert werden. Nach Ablauf dieser Zeit erfolgt dann automatisch eine Deaktivierung der Störerkennung. So entfällt die Notwendigkeit der Rückprogrammierung (Figur 1: 130).

## Patentansprüche

1. Implantierbarer Kardioverter-Defibrillator, ICD, mit einem hermetisch abgeschlossenen Gehäuse, mindestens einer Steuereinheit, mindestens einer RF-Antenne und einer RF-Kommunikationseinheit, wobei die RF-Kommunikationseinheit in Verbindung mit der RF-Antenne ausgestaltet ist, eine Kommunikation zwischen einem externen Programmiergerät und dem ICD zu ermöglichen, einem Hochspannungskondensator und einer MRT-Störerkennungseinheit mit einer Demodulationseinheit, wobei die MRT-Störerkennungseinheit mittels mindestens einer Frequenzweiche mindestens mit der RF-Kommunikationseinheit und/oder der RF-Antenne verbunden ist und die Demodulationseinheit die MRT typischen RF-Drehfelder erkennt und ein MRT-Erkennungssignal von der MRT-Störerkennungseinheit an die mindestens eine Steuereinheit übermittelt wird, **dadurch gekennzeichnet, dass** die MRT-Störerkennungseinheit nur vor oder nur beim Laden des Hochspannungskondensators eine MRT-Erkennung durchführt.

2. ICD nach Anspruch 1, **dadurch gekennzeichnet, dass** die RF-Antenne für eine Kommunikation im MICS- (medical implant communication service) Band und/oder ISM- (industrial, scientific and medical) Band ausgebildet ist.

3. ICD nach einem der vorhergehenden Ansprüche, **dadurch gekennzeichnet, dass** die MRT-Erkennung auf einer Auswertung des MRT-typischen Frequenzspektrums an der RF-Antenne beruht.

4. ICD nach einem der vorhergehenden Ansprüche, **dadurch gekennzeichnet, dass** die MRT-Störerkennungseinheit über die eine Frequenzweiche zusätzlich mit einer vorhandenen Elektrodenleitung verbunden und/oder verbindbar ist und eine MRT-Erkennung nur vorliegt, wenn RF-Drehfelder gleichzeitig sowohl über die Antenne als auch über die Elektrodenleitung erkannt werden.

5. ICD mach einem der vorhergehenden Ansprüche, **dadurch gekennzeichnet, dass** das Antennendiagramm der RF-Antenne in drei Raumrichtungen Maxima aufweist, bevorzugt in drei orthogonale Raumrichtungen.

6. ICD nach einem der vorhergehenden Ansprüche, **dadurch gekennzeichnet, dass** bei der Erkennung eines MRT-typischen Feldes in einen MRT-sicheren Zustand gewechselt wird, wobei dieser Zustand entweder bis zu einer möglichen Umprogrammierung permanent oder für einen vorbestimmten Zeitraum befristet oder bis zu einem Ausbleiben einer MRT-Erkennung oder bis zu einem Ausbleiben einer MRT-Erkennung für einen vorbestimmten Zeitraum beibehalten wird.

7. ICD nach Anspruch 5, **dadurch gekennzeichnet, dass** der MRT-sichere Zustand eine vorgegebene oder vorbestimmte Stimulationscharakteristik aufweist.

8. ICD nach Anspruch 1, **dadurch gekennzeichnet, dass** die RF-Antenne während der MRT-Erkennung auf die typischen MRT-RF-Frequenzspektren abgestimmt wird, wobei eine feste Frequenz vorgegeben sein kann oder ein oder mehrere Frequenzbereiche abgetastet werden.

9. ICD nach Anspruch 8, **dadurch gekennzeichnet, dass** die Abtastung von einem oder mehreren Frequenzbereichen entweder über einen oder mehrere Bandpassfilter erfolgt.

10. ICD nach Anspruch 1 oder 8, **dadurch gekennzeichnet, dass** das ICD für MRT-Geräte, die mit unterschiedlichen Magnetfeldstärken und damit unterschiedlichen RF-Frequenzen arbeiten, unterschiedliche vorbestimmte MRT-sichere Zustände automatisch auswählt.

11. ICD nach einem der vorangegangenen Ansprüche, **dadurch gekennzeichnet, dass** des ICD in einen MRT-sicheren Zustand geschaltet wird, wenn die MRT-Störerkennungseinheit ein MRT-Feld erkannt hat und dass der MRT-sichere Zustand eine um eine vorbestimmte Zeit verlängerbare VF-Detektion hat.

12. ICD nach einem der vorangegangenen Ansprüche, **dadurch gekennzeichnet, dass** die MRT-Störerkennungseinheit des Weiteren mit weiteren Indikatoren für MRT-Störfelder oder mindestens einem MRT-Sensor verbunden ist.

13. ICD nach einem der vorangegangenen Ansprüche, **dadurch gekennzeichnet, dass** mindestens eine der folgenden Maßnahmen bei einer MRT-Erkennung eingeleitet werden:
- das Wechseln in einen MRT-sicheren Zustand
- ein verlängertes Verbleiben in einem MRT-sicheren oder gegenüber elektromagnetischen Störfeldern unempfindlichen Zustand,
- eine Synchronisierung von elektrischen Messungen mit bei periodischen oder gepulsten elektromagnetischen Feldern auftretenden betragsmäßigen Feldstärkenminima oder die Synchronisierung einer Stimulation mit den betragsmäßigen Minima, und
- die Abgabe von elektromagnetischen Pulsen zur Signalisierung, dass ein medizinisches Gerät, speziell ein Implantat, im elektromagnetischen Feld vorhanden ist.

## Claims

1. An implantable cardioverter defibrillator (ICD), comprising a hermetically sealed housing, at least one control unit, at least one RF antenna and an RF communication unit, wherein the RF communication unit in conjunction with the RF antenna is designed to allow communication between an external programming device and the ICD, a high-voltage capacitor, and an MRT interference detection unit having a demodulation unit, wherein the MRT interference detection unit is connected at least to the RF communication unit and/or to the RF antenna by means of at least one diplexer, and the demodulation unit detects the RF rotary fields typical for MRT and transmits an MRT detection signal from the MRT interference detection unit to the at least one control unit, **characterised in that** the MRT interference detection unit performs MRT detection only before or only during charging of the high-voltage capacitor.

2. The ICD according to Claim 1, **characterised in that** the RF antenna is configured for communication in the MICS (medical implant communication service) band and/or ISM (industrial, scientific and medical) band.

3. The ICD according to one of the preceding claims, **characterised in that** the MRT detection is based on an evaluation of the frequency spectrum typical for MRT, at the RF antenna.

4. The ICD according to one of the preceding claims, **characterised in that** the MRT interference detection unit is additionally connected and/or connectable via the diplexer to an electrode line that is present, and MRT detection occurs only when RF rotary fields are simultaneously detected via the antenna and via the electrode line.

5. The ICD according to one of the preceding claims, **characterised in that** the antenna diagram of the RF antenna has maxima in three spatial directions, preferably in three orthogonal spatial directions.

6. The ICD according to one of the preceding claims, **characterised in that** a change is made to an MRT-safe state when a field typical for MRT is detected, wherein this state is either permanent until a possible reprogramming, or temporary for a predetermined period of time, or is maintained until there is no MRT detection or until there is no MRT detection for a predetermined period of time.

7. The ICD according to Claim 5, **characterised in that** the MRT-safe state has a specified or predetermined stimulation characteristic.

8. The ICD according to Claim 1, **characterised in that** during the MRT detection the RF antenna is tuned to the RF frequency spectra that are typical for MRT, wherein it is possible to specify a fixed frequency or to sample one or more frequency ranges.

9. The ICD according to Claim 8, **characterised in that** the sampling of one or more frequency ranges is carried out using one or more band pass filters.

10. The ICD according to Claim 1 or 8, **characterised in that**, for MRT devices that operate at different magnetic field intensities and thus different RF frequencies, different predetermined MRT-safe states are automatically selected by the ICD for MRT.

11. The ICD according to one of the preceding claims, **characterised in that** the ICD is switched to an MRT-safe state when the MRT interference detection unit has detected an MRT field, and **in that** the MRT-safe state has VF detection that may be prolonged by a predetermined period of time.

12. The ICD according to one of the preceding claims, **characterised in that** the MRT interference detection unit is also connected to further indicators for MRT interference fields or to at least one MRT sensor.

13. The ICD according to one of the preceding claims, **characterised in that** at least one of the following measures is introduced in the case of MRT detection:
- changing to an MRT-safe state,
- remaining for a prolonged period of time in an MRT-safe state or in a state that is insensitive to electromagnetic interference fields,
- synchronising electrical measurements using field intensity minimum absolute values occurring with periodic or pulsed electromagnetic fields, or synchronising a stimulation using the minimum values, and
- emitting electromagnetic pulses for signalling that a medical device, in particular an implant, is present in the electromagnetic field.

## Revendications

1. Défibrillateur automatique implantable, DAI, avec un boîtier fermé hermétiquement, au moins une unité de commande, au moins une antenne FR et une unité de communication FR, dans lequel l'unité de communication FR est réalisée en liaison avec l'antenne FR, afin de rendre possible une communication entre un appareil de programmation externe et le DAI, un condensateur haute tension et une unité de reconnaissance de perturbations de l'IRM avec une unité de démodulation, dans lequel l'unité de reconnaissance de perturbations de l'IRM est reliée avec l'au moins une unité de communication FR et/ou antenne FR au moyen d'au moins un filtre crossover et l'unité de démodulation reconnaît les champs tournants FR typiques de l'IRM et un signal de reconnaissance d'IRM est transmis de l'unité de reconnaissance de perturbations de l'IRM vers l'au moins une unité de commande, **caractérisé en ce que**
l'unité de reconnaissance de perturbations de l'IRM effectue une reconnaissance d'IRM uniquement avant ou pendant la charge du condensateur haute tension.

2. DAI selon la revendication 1, **caractérisé en ce que** l'antenne FR est conçue pour une communication dans la bande MICS (medical implant communication service) et/ou dans la bande ISM (industrial, scientific and medical).

3. DAI selon l'une des revendications précédentes, **caractérisé en ce que** la reconnaissance d'IRM repose sur une exploitation du spectre de fréquences typique de l'IRM sur l'antenne FR.

4. DAI selon l'une des revendications précédentes, **caractérisé en ce que** l'unité de reconnaissance de perturbations de l'IRM, par laquelle un filtre crossover est relié, et/ou peut être relié, de manière complémentaire avec une ligne d'électrode disponible et une reconnaissance d'IRM ne se présente que si des champs tournants FR sont reconnus simultanément à la fois par l'antenne et également la ligne d'électrode.

5. DAI selon l'une des revendications précédentes, **caractérisé en ce que** le diagramme d'antenne de l'antenne FR présente des valeurs maximales dans trois directions spatiales, de préférence, dans trois directions spatiales orthogonales.

6. DAI selon l'une des revendications précédentes, **caractérisé en ce que**, lors de la reconnaissance d'un champ typique d'IRM, on passe à un état sécurisé d'IRM, dans lequel cet état est soit permanent pour une reprogrammation possible, soit à durée limitée pour un laps de temps prédéterminé, ou est maintenu jusqu'à une extinction d'une reconnaissance d'IRM ou jusqu'à une extinction d'une reconnaissance d'IRM pour un laps de temps prédéterminé.

7. DAI selon la revendication 5, **caractérisé en ce que** l'état sécurisé d'IRM présente une caractéristique de stimulation prédéfinie ou prédéterminée.

8. DAI selon la revendication 1, **caractérisé en ce que** l'antenne FR est ajustée sur les spectres de fréquences RF typiques de l'IRM pendant la reconnaissance d'IRM, dans lequel une fréquence fixe peut être prédéfinie ou un ou plusieurs domaines de fréquences peuvent être testés.

9. DAI selon la revendication 8, **caractérisé en ce que** le test d'un ou de plusieurs domaines de fréquences est réalisé par l'intermédiaire soit d'un, soit de plusieurs filtres de bande passante.

10. DAI selon les revendications 1 ou 8, **caractérisé en ce que** le DAI choisit automatiquement différents états sécurisés d'IRM prédéterminés pour les appareils d'IRM, qui travaillent avec des intensités de champs magnétiques différentes et par conséquent des fréquences FR différentes.

11. DAI selon l'une des revendications précédentes, **caractérisé en ce que** le DAI est réglé dans un état sécurisé d'IRM lorsque l'unité de reconnaissance de perturbations d'IRM a reconnu un champ d'IRM, et que l'état sécurisé d'IRM a une détection de FV pouvant être prolongée pour un temps prédéterminé.

12. DAI selon l'une des revendications précédentes, **caractérisé en ce que** l'unité de reconnaissance de perturbations d'IRM est reliée de manière complémentaire avec d'autres indicateurs de champ de perturbation d'IRM ou au moins avec un capteur d'IRM.

13. DAI selon l'une des revendications précédentes, **caractérisé en ce qu'**au moins l'une des mesures suivantes est introduite lors d'une reconnaissance d'IRM :
- le passage vers un état sécurisé d'IRM
- un séjour prolongé dans un état sécurisé d'IRM ou dans un état insensible par rapport à des champs de perturbation électromagnétiques,
- une synchronisation de mesures électriques avec des valeurs minimales de champs périodiques ou électromagnétiques pulsés d'amplitudes se présentant ou la synchronisation d'une stimulation avec des valeurs minimales d'amplitudes, et
- la délivrance de pulsations électromagnétiques pour la signalisation qu'un appareil médical, particulièrement, un implant, est présent dans le champ électromagnétique.
